# EUROPÄISCHE PATENTSCHRIFT

(11) **EP 0 676 395 B1**
(45) Veröffentlichungstag und Bekanntmachung des Hinweises auf die Patenterteilung: **03.09.2003**
(21) Anmeldenummer: 95105088.9
(22) Anmeldetag: 05.04.1995
(51) Int. Cl.: C07D 207/40, C07D 333/38, C07D 307/68, C07D 401/12, C07D 409/12, C07D 405/12, C07D 409/04, C07D 405/04, C07D 403/04, C07D 403/12, A61K 31/40, A61K 31/38, A61K 31/34, A61K 31/44, A61K 31/47, A61K 31/415

(54) **Substituierte N-Heteroaroylguanidine, als Inhibitoren des zellulären Natrium-Protonen-Antiporters, als Antiarrhythmika und als Inhibitoren der Proliferation von Zellen**
Substituted N-heteroaroylguanidines, as inhibitors of sodium-hydrogen exchange, as antiarrhythmic agents and as inhibitors of the proliferation of cells
N-heteroaroylguanidines substitués, comme inhibiteurs de l'échange sodium-hydrogène, comme agents antiarrythmiques et comme inhibiteurs de la prolifération des cellules

(30) Priorität: 11.04.1994 DE 4412334
(43) Veröffentlichungstag der Anmeldung: 11.10.1995
(73) Patentinhaber: HOECHST AKTIENGESELLSCHAFT, 65929 Frankfurt am Main (DE)
(72) Erfinder: Kleemann, Heinz-Werner, Dr., D-65474 Bischofsheim (DE); Lang, Hans-Jochen, Dr., D-65719 Hofheim (DE); Schwark, Jan-Robert, Dr., D-65929 Frankfurt (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- EP-A- 0 556 672
- EP-A- 0 556 673
- EP-A- 0 556 674
- EP-A- 0 577 024
- EP-A- 0 589 336
- EP-A- 0 590 455
- EP-A- 0 622 356
- EP-A- 0 639 573
- WO-A-93/04048
- DE-A- 1 965 267
- DE-A- 2 055 727
- DE-A- 2 065 430
- FR-A- 2 001 036
- CHEMICAL ABSTRACTS, vol. 79, no. 9, 3.September 1973 Columbus, Ohio, US; abstract no. 53170t, SKVORTSOV I.M. & ALEKSASHIN Y.V. 'Furoyl- or tetrahydrofuroylguanidines' Seite 352; Spalte 2; & SU-A-380 651 (SARATOV STATE UNIVERSITY) 15.Mai 1973
- CHEMICAL ABSTRACTS, vol. 86, no. 17, 25.April 1977 Columbus, Ohio, US; abstract no. 121071h, STOLYARCHUK A.A. ET AL. 'Synthesis of furoylguanidines and comparison of their pharmacological properties with the properties of furoylureas' Seite 522; Spalte 2; & KHIM.-FARM. ZH., Bd. 10, Nr. 7, - 1976 Seiten 72-77,
- CHEMICAL ABSTRACTS, vol. 101, no. 9, 27.August 1984 Columbus, Ohio, US; abstract no. 72124v, ALEKSASHIN, Y.V. & BREGA V.D. 'Spectral characteristics of ureides, thioureides, isoselenoureides, and guanidines' Seite 593; Spalte 2; & NUKLEOFIL'NYE REAKTS. KARBONIL'NYKH SOEDIN, 1982 Seiten 98-100,
- THE JOURNAL OF MEMBRANE BIOLOGY, Bd. 120, 1991 Seiten 41-49, ESCOBALES N. & FIGUEROA J. 'Na+/Na+ exchange and Na+/H+ antiport in rabbit erythrocytes: Two distinct transport systems'
- BIOCHEMICAL AND BIOPHYSICAL RESEARCH COMMUNICATIONS, Bd. 184, Nr. 1, 15.April 1992 Seiten 112-117, SCHMID A. ET AL. 'Na+/H+ exchange in porcine cerebral capillary endothelial cells is inhibited by a benzoylguanidine derivative'
- MEDIZINISCHE KLINIK, Bd. 87, Nr. 7, 15.Juli 1992 Seiten 378-384, DÜSING R. ET AL. 'Zur klinischen Bedeutung des Na+/H+-Antiports'
- CHEMICAL ABSTRACTS, vol. 72, no. 13, 30.März 1970 Columbus, Ohio, US; abstract no. 66928b, FUJIMOTO M. 'Isoxazolylcarbonylureas' Seite 401; Spalte 1; & JP-A-44 030 268 (SHIONOGI AND CO., LTD.) 6.Dezember 1969

## Beschreibung

Substituierte N-Heteroaroylguanidine, Verfahren zu ihrer Herstellung, ihre Verwendung als Medikament oder Diagnostikum sowie sie enthaltendes Medikament Die Erfindung betrifft Heteroaroylguanidine der Formel I 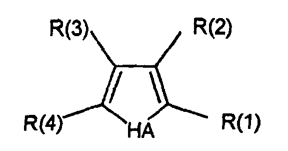 worin bedeuten:
- HA: S;
- R(1): CO-N=C(NH₂)₂;
- R(2): Wasserstoff;
- R(3): CH₃SO₂-;
- R(4): iso-Propyl, Methyl oder Br;
sowie deren pharmazeutisch verträgliche Salze.

Sie betrifft auch ein Verfahren zum Herstellen einer Verbindung I nach Anspruch 1, dadurch gekennzeichnet,
dass man Verbindungen der Formel II 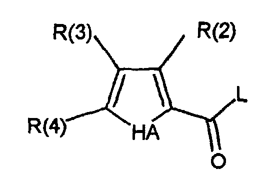 mit Guanidin umsetzt, worin HA, R(2), R(3) und R(4) wie in Anspruch 1 definiert sind und L für eine leicht nucleophil substituierbare Fluchtgruppe steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.
Neben den Carbonsäurechloriden der Formel II (L = Cl) lassen sich auch weitere aktivierte Säurederivate der Formel II in an sich bekannter Weise direkt aus den zugrundeliegenden Heteroarylcarbonsäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1,351-367 (1962)], die gemischten Anhydride II mit Cl-COOC₂H₅ oder Tosylchlorid in Gegenwart von Triethylamin in einem inerten Lösungsmittel, wie auch die Aktivierungen von Heteroarylcarbonsäuren mit Dicyclohexylcarbodiimid (DCC) oder mit O-[(Cyano(ethoxycarbonyl)-methylen)amino-1,1,3,3-tetramethyluronium-tetrafluoroborat] ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Heteroarylcarbonsäuremethylester (II, L=OMe) mit Guanidin Methanol, Isopropanol oder THF zwischen 200□□C und Siedetemperatur dieser Lösungsmittel bewährt. Bei den meisten Umsetzungen von Verbindungen II mit salzfreien Guanidin wurde vorteilhaft in inerten Lösungsmitteln wie THF, Dimethoxyethan, Dioxan oder Isopropanol gearbeitet. Aber auch Wasser kann als Lösungsmittel dienen.

Wenn L = Cl bedeutet, arbeitet man vorteilhaft unter Zusatz eines Säurefängers, z.B. in Form von überschüssigen Guanidin zur Abbindung der Halogenwasserstoffsäure.

Ein Teil der zugrundeliegenden Heteroarylcarbonsäurederivate der Formel II sind bekannt und in der Literatur beschrieben. Die unbekannten Verbindungen der Formel II können nach literatur-bekannten Methoden hergestellt werden, indem man beispielsweise 5-Halogen-4-chlorsulfonylbenzoesäuren mit Ammoniak oder Aminen in 4-Aminosulfonyl-5-Halogen-heteroarylcarbonsäuren bzw. mit einem schwachen Reduktionsmittel wie Natriumbisulfit und anschließender Alkylierung in 4-Alkylsulfonyl-5-Halogen-Heteroarylcarbonsäuren überführt und nach einer der oben beschriebenen Verfahrensvarianten zu erfindungsgemäßen Verbindungen I umgesetzt werden.

Die Einführung von substituierten Schwefel-, Sauerstoff- oder Stickstoffnucleophilen gelingt durch literaturbekannte Methoden der nucleophilen Substitution am Aromaten. Als Abgangsgruppe haben sich bei dieser Substitution Halogenide und Trifluormethansulfonate bewährt. Man arbeitet vorteilhaft in einem dipolar aprotischen Lösungsmittel, wie zum Beispiel DMF oder TMU bei einer Temperatur zwischen 0 °C und dem Siedepunkt des Lösungsmittels, bevorzugt zwischen 80°C und dem Siedepunkt des Lösungsmittels. Als Säurefänger dient vorteilhaft ein Alkali- oder Erdalkalisalz mit einem Anion hoher Basizität und geringer Nucleophilie, wie zum Beispiel K₂CO₃.

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Heteroaroylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Es war überraschend, dass die erfindungsgemäßen Verbindungen keine unerwünschten und nachteiligen salidiuretischen, jedoch sehr gute antiarrhythmische Eigenschaften aufweisen, wie sie zum Behandeln von Krankheiten wichtig sind, die beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks. Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässerige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.- %.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg, vorzugsweise 0,01 mg bis 10 mg, vorzugsweise 1 mg. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

Analog der in den Ausführungsbeispielen angegebenen Vorschriften können die nachfolgend aufgeführten erfindungsgemäßen Verbindungen der Formel I bzw. deren physiologisch verträglichen Salze hergestellt werden:

### Liste der Abkürzungen:

- MeOH: Methanol
- DMF: N,N-Dimethylformamid
- TMU: N,N,N',N'-Tetramethylharnstoff
- NBS: N-Bromsuccinimid
- AIBN: α,α-Azo-bis-isobutyronitril
- EI: electron impact
- DCI: Desorption-Chemical lonisatior
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent
- ES: Elektrospray-lonisation
- Me: Methyl
- Et: Ethyl
- Bn: Benzyl
- ZNS: Zentralnervensystem
- Brine: gesättigte wässerige NaCI-Lösung

### Experimenteller Teil

### Beispiel 1

### 5-Isopropyl-4-methylsulfonyl-thiophen-2-carbonsäureguanidid

a) 5-Brom-thiophen-2-carbonsäure
   10 g Thiophen-2-carbonsäure werden in 100 ml Essigsäure und 100 ml Wasser gelöst und bei 0 °CC eine Lösung von 4 ml Brom in 50 ml Essigsäure und 50 ml Wasser während einer Stunde zugetropft. 1 h wird bei 0 °C nachgerührt, das Produkt abgesaugt und aus Wasser umkristallisiert. Man erhält 4.8 g farbloser Kristalle, mp 140° C
   R_{f} (MTB 2 % HOAc) = 0.54 MS (DCI) : 207 (M+H)⁺
b) 5-Brom-4-chlorsulfonyl-thiophen-2-carbonsäure
   37 g 5-Brom-thiophen-2-carbonsäure werden bei RT in 133 ml Chlorsulfonsäure gelöst und bei 100 °C 45 min gerührt. Anschließend wird auf 1 kg Eis gegossen und das Produkt abgesaugt. Man erhält 53 g eines farblosen Feststoffs, mp 96° C
   R_{f} (MTB 2 % HOAc) = 0.3 MS (DCI): 305 (M+H)⁺
c) 5-Brom-4-hydroxysulfinyl-thiophen-2-carbonsäure
   27.5 g Natriumsulfit werden in 300 ml Wasser gelöst und bei 70 °C portionsweise insgesamt 35 g 5-Brom-4-chlorsulfonyl-thiophen-2-carbonsäure zugegeben, wobei mit 10 N NaOH pH = 9 - 11 gehalten wird. 2 h wird bei 70 °C nachgerührt, dann mit HCl auf pH = 1 gestellt und das Produkt abgesaugt. Man erhält 41 g farbloser Kristalle.
   mp 195 °C (Zersetzung)
d) 5-Brom-4-hydroxysulfinyl-thiophen-2-carbonsäure, Dinatriumsalz
   41 g 5-Brom-4-hydroxysulfinyl-thiophen-2-carbonsäure werden in 150 ml Wasser suspendiert und mit 90 ml 2 N NaOH versetzt (pH = 10). Das Wasser wird im Vakuum entfernt, mit 1 I Aceton verrührt und das Produkt abgesaugt. Man erhält 46 g eines farblosen, amorphen Feststoffs, der direkt weiter umgesetzt wird.
e) 5-Brom-4-methylsulfonyl-thiophen-2-carbonsäuremethylester
   46 g der Titelverbindung 1 d) werden in 150 ml DMF suspendiert und mit 32 ml Methyliodid versetzt. 5 h wird bei 50 °C gerührt, auf 1 I Wasser gegossen und das Produkt abgesaugt. Man erhält 35 g eines farblosen Feststoffs, mp 135 °C
   Rf (DIP) = 0.20 MS (DCI) : 299 (M+H)⁺
f) 5-Isopropyl-4-methylsulfonyl-thiophen-2-carbonsäuremethylester
   30 ml einer 2 M Lösung von Isopropylmagnesiumchlorid in THF werden zu 140 ml einer
   0.5 M Lösung von Zinkchlorid in THF hinzugefügt. 5 h wird bei 50 °C gerührt und das entstandene Isopropylzink-Derivat als Lösung A weiterverwendet.
   6 g 5-Brom-4-methylsulfonyl-thiophen-2-carbonsäuremethylester, 0.6 g [1,1'-Bis(diphenylphosphino)ferrocen]Pd(II)Cl₂ x CH₂Cl₂ und 180 mg Cul werden in 100 ml wasserfreiem THF 10 min bei RT gerührt und anschließend Lösung A zugetropft. 18 h wird bei RT nachgerührt und anschließend das Solvens im Vakuum entfernt. Der Rückstand wird in 200 ml gesättigter wässeriger NaHSO₄-Lösung suspendiert und 3 x mit je 200 ml EE extrahiert. Über Na₂SO₄ wird getrocknet, das Solvens im Vakuum entfernt und je einmal mit DIP und EE/HEP 1:3 chromatographiert. Man erhält 1.7 g eines farblosen Öls.
   Rf (DIP) = 0.29 Rf (EE/HEP 1:3) = 0.32 MS (DCI) : 263 (M+H)⁺
g) 5-lsopropyl-4-methylsulfonyl-thiophen-2-carbonsäureguanidid
   700 mg 5-lsopropyl-4-methylsulfonyl-thiophen-2-carbonsäuremethylester und 790 mg Guanidin werden in 5 ml wasserfreiem Isopropanol gelöst und 1 h unter Rückfluss gekocht. Das Solvens wird im Vakuum entfernt, 80 ml Wasser zugegeben, mit wässeriger HCI auf pH = 2 gestellt und das Produkt abfiltriert. Der Niederschlag wird in 50 ml gesättigter wässeriger Na₂CO₃-Lösung gelöst und 3 x mit je 50 ml EE extrahiert.
   Die organische Phase wird über Na₂SO₄ getrocknet und das Solvens im Vakuum entfernt. Man erhält 850 mg eines amorphen Feststoffs.
   R_{f} (MeOH/EE 1:10) = 0.41 MS (ES) :290 (M+H)⁺
   mp (Hydrochlorid) : 267 °C
   mp (Methansulfonat) : 128 °C

Die Titelverbindungen der Beispiele 2, 3 und 4 wurden analog Beispiel 1 g) synthetisiert:

### Beispiel 2

### 5-Methyl-thiophen-2-carbonsäureguanidid

mp (Hydrochlorid) : 236 °C MS (DCI) : 184 (M+H)⁺

### Beispiel 3

### 4,5-Dibrom-thiophen-2-carbonsäureguanidid

mp (Hydrochlorid) : 268 °C MS (DCI): 326 (M+H)⁺

### Beispiel 4

### 4-Isopropyl-5-methylsulfonyl-thiophen-2-carbonsäureguanidid

a) 4-Brom-5-methylthio-thiophen-2-carbonsäure
   25 g 4,5-Dibrom-thiophen-carbonsäure, 12,2 g NaSCH₃ und 60 g K₂CO₃ werden in 1 l DMF 5 h lang bei 120 °C gerührt. Anschließend wird auf 3 l Wasser gegossen, mit HCl auf pH = 1 gestellt, das Produkt abgesaugt und ohne Reinigung weiter eingesetzt.
   Ausbeute: 14 g amorphes Pulver.
   R_{f} (DIP 2 % HOAc) = 0.46
b) 4-Brom-5-methylsulfonyl-thiophen-2-carbonsäure
   14 g Methylthio-Verbindung 4 a) werden in 500 ml CH₂Cl₂ gelöst, und dann werden 41 g m-Chlorperbenzoesäure portionsweise zugegeben. 1.5 h wird bei RT gerührt, anschließend das Solvens im Vakuum entfernt und das Produkt ohne Reinigung verestert.
   R_{f} (DIP 2 % HOAc) = 0.10
c) 4-Brom-5-methylsulfonyl-thiophen-2-carbonsäure-methylester
   Das gesamte Rohprodukt des Beispiels 4 b) wird in 200 ml MeOH mit 50 ml SOCl₂ versetzt und 5 h unter Rückfluss gekocht. Überschüssiges SOCl₂ sowie das Solvens werden im Vakuum entfernt und der Rückstand mit DIP chromatographiert. Man erhält 11 g eines farblosen Öls.
   Rf (DIP) = 0.28 MS (DCI) : 299 (M+H)⁺
d) 4-Isopropyl-5-methylsulfonyl-thiophen-2-carbonsäure-methylester
   30 ml einer 2 M lsopropylmagnesiumchlorid-Lösung in Diethylether werden zu einer 1 M Lösung von ZnCl₂ in Diethylether zugetropft und 6 h unter Rückfluss gekocht. (Lösung A)
   6 g Bromid 4 c), 588 mg [1,1-Bis(diphenylphosphino)ferrocen]Pd(II)Cl₂ und 183 mg Cul werden in 100 ml THF 10 min. bei RT gerührt und anschließend mit Lösung A versetzt. 19 h wird bei RT gerührt, 200 ml EE zugegeben und je 1 x mit 200 ml Wasser und 200 ml Brine gewaschen. Das Solvens wird im Vakuum entfernt und mit EE/HEP 1:2 chromatographiert.
   Man erhält 2 g eines farblosen Öls.
   R_{f}(EE/HEP 1:2) = 0.25 MS (DCI): 263 (M+H)⁺
e) 4-Isopropyl-5-methylsulfonyl-thiophen-2-carbonsäureguanidid
   1 g Methylester 4 d) werden analog Beispiel 1 g) mit 1.1 g Guanidin umgesetzt. Man erhält 900 mg eines amorphen Pulvers.
   Rf(EE/MeOH 10:1) = 0.41 MS (ES): 290 (M+H)⁺
Die Verbindung wird in das Methansulfonat überführt. mp = 210° C

### Beispiel 5

### 3-Methyl-thiophen-2-carbonsäureguanidid

mp (Hydrochlorid) : 232 °C MS (DCI): 184 (M+H)⁺
Pharmakologische Daten:
Inhibition des Na⁺/H⁺-Exchangers von Kaninchenerythrocyten

Weiße Neuseeland-Kaninchen (Ivanovas) erhielten eine Standard-Diät mit 2 % Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCI, 150 Sucrose, 0,1 Ouabain, 20 Tris-hydroxymethyl-aminomethan) bei pH 7,4 und 37 °C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀ [µmol/l] |
| 1 | 0.5 |
| 2 | 3 |
| 3 | 0.5 |

## Patentansprüche

1. Guanidine der Formel I 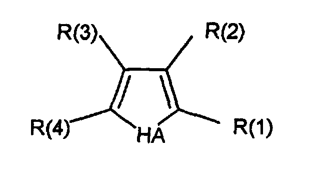 worin bedeuten:
HA S;
R(1) CO-N=C(NH₂)₂;
R(2) Wasserstoff;
R(3) CH₃SO₂-;
R(4) iso-Propyl, Methyl oder Br;
sowie deren pharmazeutisch verträgliche Salze.

2. Verfahren zum Herstellen einer Verbindung I nach Anspruch 1, **dadurch gekennzeichnet,**
**dass** man Verbindungen der Formel II 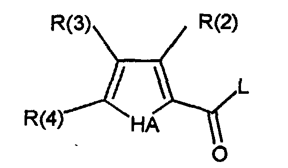 mit Guanidin umsetzt, worin HA, R(2), R(3) und R(4) wie in Anspruch 1 definiert sind und L für eine leicht nucleophil substituierbare Fluchtgruppe steht.

3. Verwendung einer Verbindung I nach Anspruch 1 zur Herstellung eines Medikaments zu Behandlung von Arrhythmien.

4. Verwendung einer Verbindung I nach Anspruch 1 zur Herstellung eines Medikaments zur Behandlung oder Prophylaxe des Herzinfarkts.

5. Verwendung einer Verbindung I nach Anspruch 1 zur Herstellung eines Medikaments zur Behandlung oder Prophylaxe der Angina Pectoris.

6. Verwendung einer Verbindung I nach Anspruch 1 zur Herstellung eines Medikaments zur Behandlung oder Prophylaxe von ischämischen Zuständen des Herzens.

7. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

8. Verwendung einer Verbindung I nach Anspruch 1 zur Herstellung eines Medikaments zur Behandlung oder Prophylaxe von ischämischen Zuständen peripherer Organe und Gliedmaßen.

9. Verwendung einer Verbindung I nach Anspruch 1 zur Herstellung eines Medikaments zur Behandlung von Schockzuständen.

10. Verwendung einer Verbindung I nach Anspruch 1 zur Herstellung eines Medikaments zum Einsatz bei chirurgischen Operationen und Organtransplantationen.

11. Verwendung einer Verbindung I nach Anspruch 1 zur Herstellung eines Medikaments zur Konservierung und Lagerung von Transplantaten für chirurgische Maßnahmen.

12. Verwendung einer Verbindung I nach Anspruch 1 zur Herstellung eines Medikaments zur Behandlung von Krankheiten, bei denen die Zellproliferation eine primäre oder sekundäre Ursache darstellt.

13. Heilmittel, enthaltend eine wirksame Menge einer Verbindung I nach Anspruch 1.

## Claims

1. A guanidine of the formula I 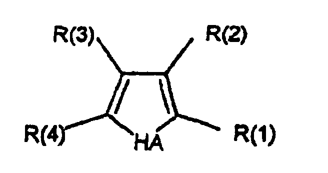 in which:
HA is S;
R(1) is CO-N=C(NH₂)₂;
R(2) is hydrogen;
R(3) is CH₃SO₂-;
R(4) is isopropyl, methyl or Br;
and the pharmaceutically tolerated salts thereof.

2. A process for preparing a compound I as claimed in claim 1, wherein
a compound of the formula II 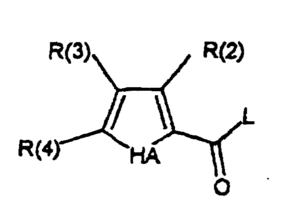 in which HA, R(2), R(3) and R(4) are as defined in claim 1 and L is a leaving group which can readily be substituted nucleophilically, is reacted with guanidine.

3. The use of a compound I as claimed in claim 1 for preparing a medicament for the treatment of arrhythmias.

4. The use of a compound I as claimed in claim 1 for preparing a medicament for the treatment or prophylaxis of cardiac infarction.

5. The use of a compound I as claimed in claim 1 for preparing a medicament for the treatment or prophylaxis of angina pectoris.

6. The use of a compound I as claimed in claim 1 for preparing a medicament for the treatment or prophylaxis of ischemic conditions of the heart.

7. The use of a compound I as claimed in claim 1 for preparing a medicament for the treatment or prophylaxis of ischemic conditions of the peripheral and central nervous system and of stroke.

8. The use of a compound I as claimed in claim 1 for preparing a medicament for the treatment or prophylaxis of ischemic conditions of peripheral organs and limbs.

9. The use of a compound I as claimed in claim 1 for preparing a medicament for the treatment of shock conditions.

10. The use of a compound I as claimed in claim 1 for preparing a medicament for employment in surgical operations and organ transplantations.

11. The use of a compound I as claimed in claim 1 for preparing a medicament for the preservation and storage of transplants for surgical procedures.

12. The use of a compound I as claimed in claim 1 for preparing a medicament for the treatment of diseases in which cell proliferation represents a primary or secondary cause.

13. A medicine containing an effective quantity of a compound I as claimed in claim 1.

## Revendications

1. Guanidines de formule I 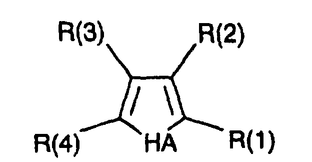 où :
HA représente S ;
R(1) représente CO-N=C(NH₂)₂ ;
R(2) représente l'hydrogène ;
R(3) représente CH₃SO₂- ;
R(4) représente isopropyle, méthyle ou Br ;
ainsi que leurs sels pharmaceutiquement acceptables.

2. Procédé de préparation d'un composé I selon la revendication 1, **caractérisé en ce que** l'on fait réagir des composés de formule II 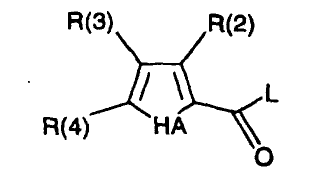 avec la guanidine, où HA, R(2), R(3) et R(4) sont définis comme dans la revendication 1 et L représente un groupe partant aisément substituable par substitution nucléophile.

3. Utilisation d'un composé I selon la revendication 1 pour la préparation d'un médicament pour le traitement des arythmies.

4. Utilisation d'un composé I selon la revendication 1 pour la préparation d'un médicament pour le traitement ou la prophylaxie de l'infarctus du myocarde.

5. Utilisation d'un composé I selon la revendication 1 pour la préparation d'un médicament pour le traitement ou la prophylaxie de l'angine de poitrine.

6. Utilisation d'un composé I selon la revendication 1 pour la préparation d'un médicament pour le traitement ou la prophylaxie des états ischémiques du coeur.

7. Utilisation d'un composé I selon la revendication 1 pour la préparation d'un médicament pour le traitement ou la prophylaxie des états ischémiques du système nerveux périphérique et central et de l'apoplexie cérébrale.

8. Utilisation d'un composé I selon la revendication 1 pour la préparation d'un médicament pour le traitement ou la prophylaxie des états ischémiques des organes périphériques et des membres.

9. Utilisation d'un composé I selon la revendication 1 pour la préparation d'un médicament pour le traitement des états de choc.

10. Utilisation d'un composé I selon la revendication 1 pour la préparation d'un médicament destiné à être utilisé dans des opérations chirurgicales et des greffes d'organes.

11. Utilisation d'un composé I selon la revendication 1 pour la préparation d'un médicament pour la conservation et le stockage de greffons pour opérations chirurgicales.

12. Utilisation d'un composé I selon la revendication 1 pour la préparation d'un médicament pour le traitement des maladies dans lesquelles la prolifération cellulaire constitue une cause primaire ou secondaire.

13. Remède contenant une quantité efficace d'un composé I selon la revendication 1.
